(19) **Europäisches Patentamt / European Patent Office / Office européen des brevets**

(11) **EP 4 707 787 A1**

(12) # EUROPEAN PATENT APPLICATION

(43) Date of publication:
**11.03.2026 Bulletin 2026/11**

(21) Application number: **24315415.0**

(22) Date of filing: **10.09.2024**

(51) International Patent Classification (IPC):
**G01N 23/2055** (2018.01)    **G16C 60/00** (2019.01)

(52) Cooperative Patent Classification (CPC):
**G01N 23/2055**; G16C 20/70; G16C 60/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Altrove Tech**
**75008 Paris (FR)**

(72) Inventor: **LAULAINEN, Joonatan**
**Paris (FR)**

(74) Representative: **Mathys & Squire**
**Theatinerstraße 7**
**80333 München (DE)**

(54) **SYSTEMS AND METHODS FOR MATERIALS CHARACTERISATION**

(57)     A computer implemented method (100) of characterising a crystalline material is disclosed. The method (100) includes obtaining diffraction data (101) and a predicted phase distribution (11) from a crystalline material. Modified Rietveld refinement is performed on the diffraction data. This includes i) determining an R-weighted pattern value, $R_{wp}$, and an R-expected value, $R_e$, for a structural model of the crystalline material and ii) determining (104, 107) a goodness-of-fit value based on $R_{wp}$, $R_e$ and a likelihood estimator for the structural model. The goodness-of-fit value is optimised (108) by repeating steps i) and ii) for different structural models of the crystalline material. An optimised structural model that provides an optimised goodness-of-fit value is obtained and the method outputs (109) a characterisation of the crystalline material based on the obtained optimised structural model. The likelihood estimator is based on the predicted phase distribution (11) of the crystalline material and a measured phase distribution (105), which is based on the structural model.

FIG. 1

EP 4 707 787 A1

**Description**

<u>Field of the invention</u>

The present disclosure relates to systems and methods in the field of materials characterisation, in particular to analysing diffraction patterns using Rietveld refinement.

<u>Background</u>

Rietveld refinement is a technique used for the characterisation and identification of crystalline materials. It is a non-linear least squares method that aims to minimise the difference between an experimental and calculated profile, and uses this to refine structural (e.g. unit cell size, atomic positions, strain) and instrumental (e.g. angular dispersion, wavelength) parameters. It is a multi-step process that requires expert input, in particular regarding the order in which to fit parameters. Most new materials are structurally characterised using X-ray diffraction (X-ray diffraction), either alone or in combination with other techniques.

Typically, XRD crystallography is used to measure characteristics of crystalline materials, such as the positions of atoms in a crystal structure, from which the crystallographic phase can be determined. Once a diffraction pattern has been obtained, it can be analysed by comparing the measured pattern to expected diffraction patterns of known materials. For example, the position and intensities of diffraction peaks can be compared between the measured pattern and one or more expected patterns. This can be done computationally using a Rietveld refinement method. For example, based on an initial prediction of the phase of the material, an expected diffraction pattern can be calculated. The calculated pattern can be compared to the measured pattern to determine a goodness-of-fit.

The Rietveld refinement algorithm R-factor, $R_{wp}$, which is minimised for a fit, is typically written as a weighted sum:

$$R_{wp} = \left( \frac{\sum w_i (y_i - y_{ci})^2}{\sum w_i y_i^2} \right)^{1/2}$$

where $w_i$ are the weights of data points $y_i$ and are equal to $1/\sigma_i^2$ where $\sigma_i$ is the standard error of data point $y_i$, and $y_{ci}$ is the predicted and computed value of $y_i$ based on the structural model.

Typically, the reported quality metric is the goodness-of-fit $S$ which is defined as:

$$S = \frac{R_{wp}}{R_e}$$

where $R_e$ is sometimes called R-expected and is the value of $R_{wp}$ for a perfect model (and so is only dependent on the level of noise in the data in $y_i$), written as follows:

$$R_e = \left[ \frac{(N - P)}{\sum w_i y_i^2} \right]^{1/2}$$

where $N$ is the number of data points and P is the number of parameters.

By iteratively adjusting the parameters of the model (e.g. atomic positions, lattice parameters etc.), multiple candidate models can be evaluated by calculating the goodness-of-fit and refining the model until the goodness-of-fit has been optimised (i.e., minimised). This method can be highly complex for mixtures of materials and cannot generally be automated due to the complexity of fitting multiple related parameters, such as the background, atomic occupancy, strain, peak shape, temperature, sample orientation, and lattice parameter.

It is an aim of the present invention to improve automated analysis and optimisation of materials systems using automated Rietveld refinement.

<u>Summary of the invention</u>

Aspects of the disclosure are set out in the independent claims and optional features are set out in the dependent claims. Aspects of the disclosure may be provided in conjunction with each other, and features of one aspect may be applied to other aspects.

An aspect of the disclosure provides a computer implemented method of characterising a crystalline material, the method comprising any or all of the following steps: obtaining diffraction data from diffraction of the crystalline material; obtaining a predicted phase distribution of the crystalline material; performing modified Rietveld refinement on the diffraction data, wherein performing modified Rietveld refinement comprises: i) determining an R-weighted pattern value, $R_{wp}$, and an R-expected value, $R_e$, for a structural model of the crystalline material; and ii) determining a goodness-of-fit value based on $R_{wp}$, $R_e$ and a likelihood estimator for the structural model; optimising the goodness-of-fit value by repeating steps i) and ii) for different structural models of the crystalline material; obtaining an optimised structural model that provides an optimised goodness-of-fit value; outputting a characterisation of the crystalline material based on the obtained optimised structural model; wherein the likelihood estimator is based on: the predicted phase distribution of the crystalline material; and a measured phase distribution, wherein the measured phase distribution is based on the structural model.

Obtaining the predicted phase distribution of the crystalline material may be performed before the step of performing modified Rietveld refinement. Obtaining the predicted phase distribution may be based on information of the crystalline material. The information may be available before the step of performing modified Rietveld refinement. For example, the predicted phase distribution may be based on synthesis information of the crystalline material. The predicted phase distribution may be based on at least one of: ratios of precursor compositions; thermodynamic stability of precursor compositions; reaction temperature; reaction time; reaction atmosphere; reaction heating and cooling rates; applied fields; and instrumentation. The diffraction data may be from X-ray diffraction of the crystalline material.

Obtaining a predicted phase distribution may be performed using a different method than Rietveld refinement.

The predicted phase distribution may be based on at least one of: a graph network, an autoencoder, and a regression algorithm. The predicted phase distribution may comprise fractions of expected phases.

The predicted phase distribution may be based on a machine learning model. The machine learning model may be trained to predict a phase distribution based on input data comprising synthesis information.

The goodness-of-fit value may be based on a ratio of $R_{wp}$ to $R_e$. The goodness-of-fit value may be based on adding the likelihood estimator to the ratio. The likelihood estimator may be based on an error between the predicted phase distribution and the measured phase distribution. The likelihood estimator may be based on at least one of: a root mean square error metric and a Kullback-Leibner divergence metric.

An aspect of the disclosure provides a computer program product comprising program instructions configured to program a programmable processor to perform the method as described hereinabove.

An aspect of the disclosure provides programmable logic configured to perform the method described hereinabove.

Brief description of the drawings

Embodiments of the disclosure will now be described, by way of example only, with reference to the accompanying drawings, in which:

> Fig. 1 shows a flowchart of a method according to embodiments of the disclosure;
> Fig. 2 is a table illustrating an example calculation performed by the method of Fig. 1;
> Fig. 3A is a fitted diffraction pattern of a first example structural model;
> Fig. 3B is a fitted diffraction pattern of a second example structural model; and
> Fig. 4 is a schematic illustration of a computer system for implementing the method of Fig. 1.

Detailed description of the drawings

Embodiments of the disclosure relate to a computer implemented method of characterising a material. The method includes obtaining a measured diffraction pattern of a crystalline material to be characterised. Rietveld refinement is then performed based on the measured diffraction pattern and an initial model of the crystalline material. Additional information of the crystalline material is also obtained, such as a prediction of the phase distributions in the crystalline material which may be in the form of a volumetric ratio of phases. Such a prediction may be calculated based on the synthesis conditions of the material (e.g. reaction times and temperatures), and may be performed manually, for example using phase diagrams, or automatically, for example using software packages.

The predicted phase distribution is taken into account by the Rietveld refinement method. In particular, a goodness-of-fit value is calculated not only based on the R-factor, $R_{wp}$, and the R-expected, $R_e$, but also based on an additional term calculated from the predicted phase distribution. For example, the additional term may be calculated based on a root mean square error between a measured phase distribution (e.g., the phase distribution of the model used in Rietveld refinement) and a predicted phase distribution (e.g., the phase distribution predicted based on the reaction conditions). As such, the additional term can be considered a likelihood estimator because it evaluates how likely the predicted model used in Rietveld refinement is to exist based on the reaction conditions. If the error is greater, then the likelihood estimator will bias the goodness-of-fit to a higher value (i.e., a worse fit). This has the effect that, for two models being evaluated using Rietveld refinement which may have similar goodness-of-fit values, the model which is more likely to exist based on the reaction conditions will be considered by the Rietveld method as a better candidate for the measured diffraction pattern, such that the Rietveld refinement is more likely to converge on a structural model that is more likely to exist.

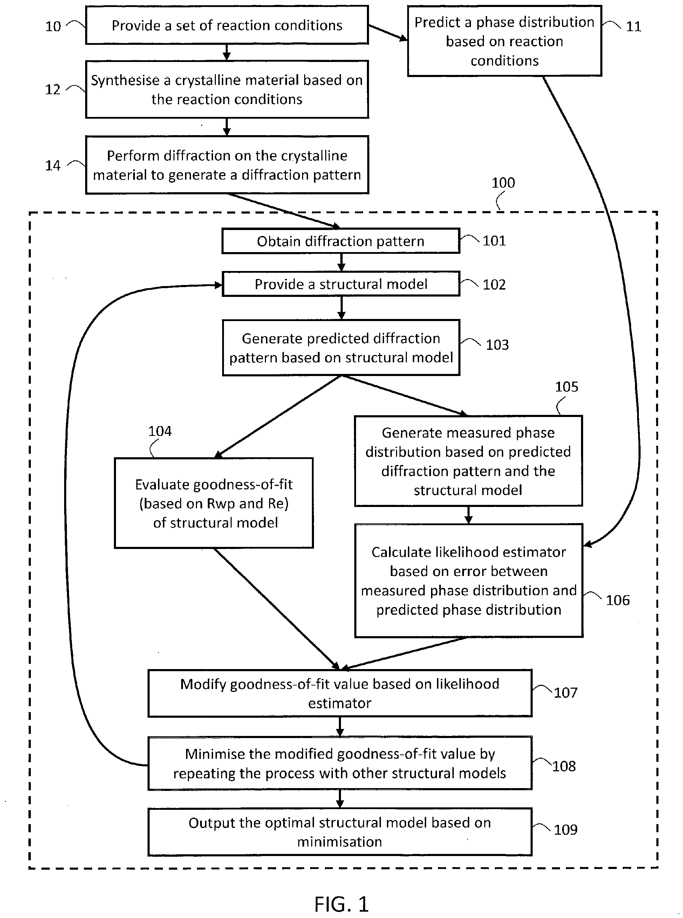

Figure 1 illustrates a method 100. The method 100 is a computer-implemented method of characterising a crystalline material. Such characterisation of a crystalline material may involve characterising particular properties of the crystalline material, such as the composition, phase distributions, grain size, lattice parameters, atomic positions and strain.

The method 100 uses Rietveld refinement as a basis for characterising a crystalline material. As such, the method 100 comprises obtaining 101 a diffraction pattern. The diffraction pattern may be in any form, such as a .xy or .rasx format. In the example shown, a diffraction pattern of a crystalline material is obtained by the following steps.

Firstly, a set of reaction conditions is provided 10. The reaction conditions may include one or more of the following: compositions of the precursors; ratios of the precursors; reaction temperature; reaction time; reaction atmosphere; reaction heating and/or cooling rates; applied electric field; and applied magnetic field. It will be appreciated that additional reaction conditions may be specified.

Secondly, a crystalline material is synthesised 12 based on the reaction conditions. This may include combining a specified ratio of precursors in a rection chamber and subjecting the mixture to the reaction conditions specified in the previous step 10, such as by heating the mixture to a specified temperature at a specified rate, before cooling the mixture to a specified temperature at a specified rate. Overall, the step of synthesising 12 the crystalline material is configured to create a crystalline material based on the reaction conditions.

Finally, diffraction is performed 14 on the crystalline material to generate a diffraction pattern. It will be appreciated by the skilled person that various sample preparation steps may be required before performing diffraction 14 on the crystalline material. After these steps have been performed, diffraction may be performed for example using a diffractometer, such as one arranged using a Bragg-Brentano geometry. As such, the diffraction data obtained by performing 14 diffraction on the crystalline material may be X-ray diffraction data.

Wherever the measured diffraction pattern has originated from, the method receives 101 the diffraction pattern for use in the method 100. This may be achieved by importing a file representing the diffraction pattern into a software package, for example.

The method 100 comprises providing 102 a structural model of the crystalline material. The structural model may be in the form of .cif or .csv files. The structural model may include information including, for example, atomic positions, lattice parameters, space groups, and so on. In some examples, providing 102 the structural model may be performed manually by a user inputting a structural model, for example by importing a file representing the structural model into a software package. In some examples, this step may be performed automatically by the method 100. Whether this step 102 is performed automatically or manually, providing 102 the structural model may involve a database lookup. For example, the diffraction pattern obtained in the previous step 101 may be analysed and compared to diffraction patterns of known compounds which may be stored in a database. This step 102 may be configured to select a known diffraction pattern that is similar to the measured diffraction pattern. Then, the step 102 may obtain information (which may also be stored in the database) from the compound which generated the diffraction pattern, and use this information to provide 102 the structural model.

The method 100 comprises generating 103 a predicted diffraction pattern based on the structural model. Generating 103 the predicted diffraction pattern may include using a known diffraction pattern from a database. For example, where a structural model is provided 102 by comparing the measured diffraction pattern to a database of known diffraction patterns, generating 103 the predicted diffraction pattern may comprise using the known diffraction pattern. In some examples, this step 103 may include using known algorithms for predicting a diffraction pattern based on a provided structural model. For example, where a user manually provides a structural model (e.g., by importing a file), the step 103 may be configured to generate a predicted diffraction pattern based on that provided structural model 102.

After the step of generating 103 the predicted diffraction pattern, it will be appreciated that the method 100 has obtained a measured diffraction pattern (e.g., measured from the synthesised crystalline material) and a predicted diffraction pattern (e.g., from the structural model). The method 100 is configured to evaluate how well the predicted diffraction pattern fits with the measured diffraction pattern. This is performed by evaluating 104 a goodness-of-fit ('S') of the structural model, for example as explained in the background section. The S value is obtained by determining an R-weighted pattern value, $R_{wp}$. This can be calculated using the following equation:

$$R_{wp} = \left( \frac{\sum w_i (y_i - y_{ci})^2}{\sum w_i y_i^2} \right)^{1/2}$$

where $w_i$ are the weights of data points $y_i$, and are equal to $1/\sigma_i^2$, where $\sigma_i$ is the standard error of data point $y_i$, and $y_{ci}$ is the predicted and computed value of $y_i$ based on the structural model. As such, $R_{wp}$ may be calculated based on the error between the intensities ($y_i$) of the measured diffraction pattern and the intensities ($y_{ci}$) of the predicted diffraction pattern for each diffraction angle ($i$). In particular, $R_{wp}$ may be calculated based on the sum of squares of such errors. The sum of squares of the errors may be weighted by a weighting factor ($w_i$). The weighting factor for each diffraction angle may be based on the standard error of the data point of the measured diffraction pattern. The weighting factor may be inversely proportional to the square of the standard error.

The S value also depends on the R-expected, $R_e$, which can be calculated as follows:

$$R_e = \left[ \frac{(N - P)}{\sum w_i y_i^2} \right]^{1/2}$$

where $N$ is the number of data points and $P$ is the number of parameters. As such, $R_e$ may be calculated based on a difference between the number of data points and the number of parameters. The number of parameters may be based on the structural model, such as lattice parameters and peak shape parameters, as well as a chosen background model.

Based on $R_e$ and $R_{wp}$, the step 104 is configured to calculate an S value. This may be defined as:

$$S = \frac{R_{wp}}{R_e}.$$

In other words, the S value may be based on a ratio of $R_{wp}$ to $R_e$. The S value therefore represents the goodness-of-fit of the structural model based purely on the predicted diffraction pattern. In a typical Rietveld refinement method, the method may loop at this stage by trying new structural models (e.g. by trying new lattice parameters and observing the change in the S value) and evaluating the goodness-of-fit value for each structural model with a view to optimising (e.g. by minimising) the goodness-of-fit.

However, in the present disclosure, the method 100 comprises a step of modifying 107 the S value based on a likelihood estimator. The likelihood estimator may represent how likely a given structural model is to exist. The likelihood estimator is based on a predicted phase distribution and a measured phase distribution. In each case, it will be appreciated that a phase distribution is a distribution of the phases present in the crystalline material, which may be in the form of phase fractions (e.g., by volume or by weight).

The method comprises generating 105 a measured phase distribution. The step of generating 105 the measured phase distribution is based on the structural model. In the example shown, the step of generating the measured phase distribution is based on the predicted diffraction pattern and the structural model. In other words, the method 100 is configured to use information from the predicted diffraction pattern and/or the structural model to generate a measured phase distribution. The measured phase distribution may be calculated by a first pass of Rietveld refinement. In some examples, the measured phase distribution is calculated by other computational algorithms, such as machine learning methods.

The predicted phase distribution is a predicted phase distribution of the crystalline material. The predicted phase distribution may be based on at least one of: a graph network, an autoencoder, and a regression algorithm. The predicted phase distribution can be based on synthesis information of the crystalline material. As such, the predicted phase distribution may be obtained based on the reaction conditions. The predicted phase distribution may be based on at least one of: ratios of precursor compositions; thermodynamic stability of precursor compositions; reaction temperature; reaction time; reaction atmosphere; reaction heating and cooling rates; applied fields; and instrumentation. This is shown in Figure 1 by the step of predicting 11 a phase distribution based on the step of providing 10 a set of reaction conditions. The prediction step 11 may be performed manually, for example by a user of the method 100. The prediction step 11 may be performed algorithmically, for example using known algorithms for predicting a phase distribution based on a set of reaction conditions. In the illustrated example, the prediction step 11 occurs outside of the method 100. As such, the method 100 is configured to receive a prediction of the phase distribution (e.g. by importing a file representative of the phase distribution). In other examples, the method 100 is configured to receive information of the synthesis conditions and generate a prediction of the phase distribution as part of the method 100. The prediction of the phase distribution may be based on a machine learning model. The machine learning model may be trained to predict a phase distribution based on input data comprising synthesis information of the crystalline material.

The inventors have recognised that comparing these distributions can indicate how likely a particular compound is to exist. For example, if the phase distributions are significantly different, then it is unlikely that the particular structural model is accurate, even if the predicted diffraction pattern happens to indicate a good fit.

Therefore, the method 100 comprises a step of calculating 106 the likelihood estimator based on an error between the measured phase distribution and the predicted phase distribution. The distributions can be compared using a number of methods. In an example, a root mean square error method is used to compare the predicted phase distribution with the measured phase distribution. An example calculation for a likelihood estimator, $l_p$, is provided below:

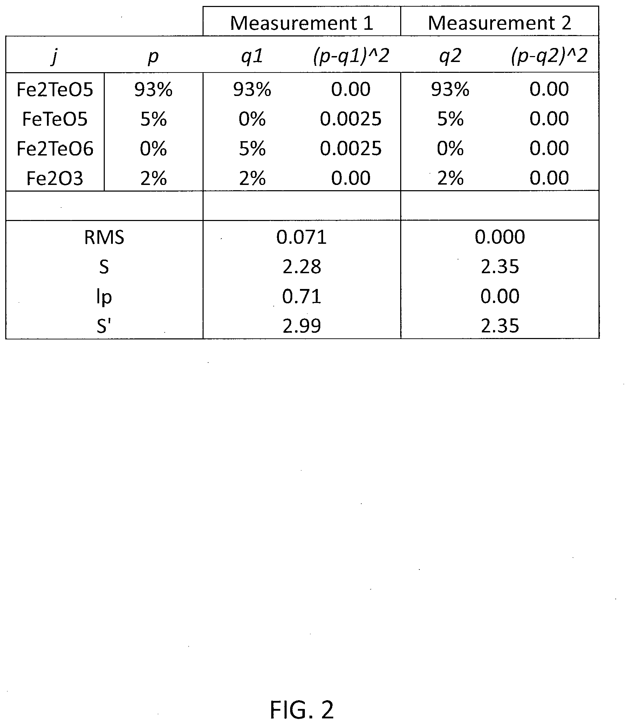

$$l_p = k \left( \sum_j \left( q_j - p_j \right)^2 \right)^{1/2}$$ where $q_j$ is the measured phase fraction and $p_j$ is the predicted phase fraction of compound $j$, and the sum is over all compounds present in the measured phase distribution and the predicted phase distribution, and $k$ is a scaling factor. Therefore, for each phase, the phase fractions present in the predicted and measured distributions are compared by calculating their squared difference, summing the squared difference for each phase, and calculating a square root of the summation. The likelihood estimator may also include a scale factor. An example of this is outlined later with reference to Figure 2. The scale factor may be chosen by various methods, such as: expert input (e.g. by providing a predefined scale factor); hyperparameter tuning, i.e. using data with known solutions and testing which scale factors result in good convergence; and/or by evaluating the effect of the scale factor on the goodness-of-fit value.

In another example, the likelihood estimator is calculated using a Kullback-Leibler Divergence measure:

$$l_p = KL(q||p) = \sum_j q_j \log \frac{q_j}{p_j}$$

where $q_j$ is the measured phase fraction and $p_j$ is the predicted phase fraction of compound j.

In any case, the likelihood estimator is calculated such that a lower likelihood estimator indicates a measured phase distribution that is closer to the predicted phase distribution, such that the structural model from which the measured phase distribution was generated has a greater chance of existence based on the reaction conditions.

The method 100 comprises modifying 107 the goodness of fit value based on the likelihood estimator. This step 107 may comprise adding the likelihood estimator to the S value to obtain a modified goodness-of-fit value, S':

$$S' = S + l_p = \frac{R_{wp}}{R_e} + l_p.$$

Given that, as explained above, the modified Rietveld refinement method aims to minimise the goodness-of-fit value, it will be appreciated in the context of the present disclosure that by adding a likelihood estimator which becomes smaller for a more accurate phase distribution prediction, the modified goodness-of-fit value S' is biased towards compounds which have a greater likelihood of existing based on the reaction conditions.

The method 100 comprises minimising 108 the modified goodness-of-fit value. This may be performed by repeating the process with other structural models. In the illustrated example, repeating the process may comprise repeating steps 102-107. The method 100 may comprise providing a structural model and obtaining, using steps 103-107 for example, a modified goodness-of-fit value, and comparing the modified goodness-of-fit value with previous calculated values. The method 100 can therefore minimise the modified goodness-of-fit value by selecting new structural models which are closer to those which resulted in the lowest modified goodness-of-fit values. New structural models may be provided based on known methods typically performed in Rietveld refinement. For example, the method 100 may successively evaluate structural models based on an incremental change in a first lattice parameter, before evaluating structural models based on an incremental change in a second lattice parameter, and so on. Eventually, as with known implementations of automated Rietveld refinement methods, there will reach a point when no further refinement of the structural model is possible and a minimum goodness-of-fit value has been reached. At this point, the method 100 has generated an optimised structural model that provides an optimised (i.e., minimised) goodness-of-fit value.

The method 100 comprises outputting 109 the optimised structural model based on the minimisation. This can provide the method's best estimation of a characterisation of the crystalline material.

## Worked Example

An example of the implementation of the method 100 of Figure 1 will now be described with reference to a reaction between the compounds Fe2O3 and TeO2.

Providing 10 a set of reaction conditions comprises providing the following reaction conditions: combining 51% $Fe_2O_3$ and 49% $TeO_2$ in a furnace at 700°C for 14 hours under an $O_2$-atmosphere.

Predicting 11 a phase distribution based on the reaction conditions may determine algorithmically that the crystalline material comprises 93% $Fe_2TeO_5$ with 5% $FeTeO_5$ and 2% leftover $Fe_2O_3$. Synthesising 12 a crystalline material based on the reaction conditions comprises executing the reaction conditions to obtain a crystalline material. Performing 14 diffraction on the crystalline material may comprise using an X-ray diffractometer to generate a diffraction pattern of the crystalline material.

The method 100 then obtains 101 the diffraction pattern generated in the previous step 14. Based on a database comparison, a first structural model is provided 102, which in this example suggests that $Fe_2TeO_5$ is the primary component. The structural model is refined using a method such as Rietveld refinement to adjust the exact fractions of the different components. The method 100 then includes evaluating 104 the goodness-of-fit by calculating Rwp and Re of the predicted diffraction patten compared to the measured diffraction pattern. In this example, the calculated S value is 2.28

The method 100 also includes generating 105 a measured phase distribution based on the predicted diffraction pattern and the structural model. In this example, the first measured phase distribution (based on the first structural model) is 93% $Fe_2TeO_5$ with 5% $Fe_2TeO_6$ and 2% leftover $Fe_2O_3$. As such, compared to the predicted phase distribution of the crystalline material, the measured phase distribution identifies $Fe_2TeO_6$ instead of $FeTeO_5$.

The method 100 then includes calculating 106 a likelihood estimator based on an error between the measured phase distribution and the predicted phase distribution. The goodness-of-fit value evaluated in step 104 is then modified 107 based on the likelihood estimator, for example by summing the goodness-of-fit value and the likelihood estimator.

The method 100 then includes minimising 108 the goodness-of-fit value by repeating the process with other structural models. For example, a second structural model may have slightly different lattice parameters than the first structural model. Steps 102-107 are repeated for different structural models, and a modified goodness-of-fit value is calculated each time with a view to identifying a minimised goodness-of-fit value. Once the refinement cannot minimise the goodness-of-fit further, the method 100 outputs 109 the optimal structural model.

Figure 2 illustrates an example calculation of the likelihood estimator using the root mean square error method discussed above. The first column ($j$) includes all the compounds from the predicted phase distribution and the measured phase distribution. The second column (p) provides the predicted phase distributions, as outlined above with reference to step 11 of the example implementation. The first structural model discussed above provides a first measured phase distribution ('Measurement 1') with the phase distributions provided above with reference to step 105 of the example implementation - these are provided in the third column ($q1$). The fourth column (($p-q1$)^2) provides the squared errors between $p$ and $q1$ for each phase fraction $j$.

The table in Figure 2 also contains phase distribution information for another measured phase distribution, which for convenience of terminology is referred to as a second measured phase distribution ('Measurement 2'). The fifth column ($q2$) provides the phase distributions of the second measured phase distribution and the sixth column (($p-q2$)*2) provides the squared errors between $p$ and $q2$ for each phase fraction $j$.

The second measured phase distribution may be generated based on a structural model, which for convenience of terminology will be referred to as a second structural model. For the avoidance of doubt, it is noted that the second structural model is not necessarily the structural model which is provided sequentially after the first structural model. Rather, there may be other structural models generated and evaluated between the first and second structural models. In this example, it is considered that the S value of the second structural model evaluated by step 104 of the method is 2.35. Therefore, compared to the S value of 2.28 for the first structural model, it might appear that the first structural model is more accurate based on the fit of the predicted diffraction pattern with the measured diffraction pattern.

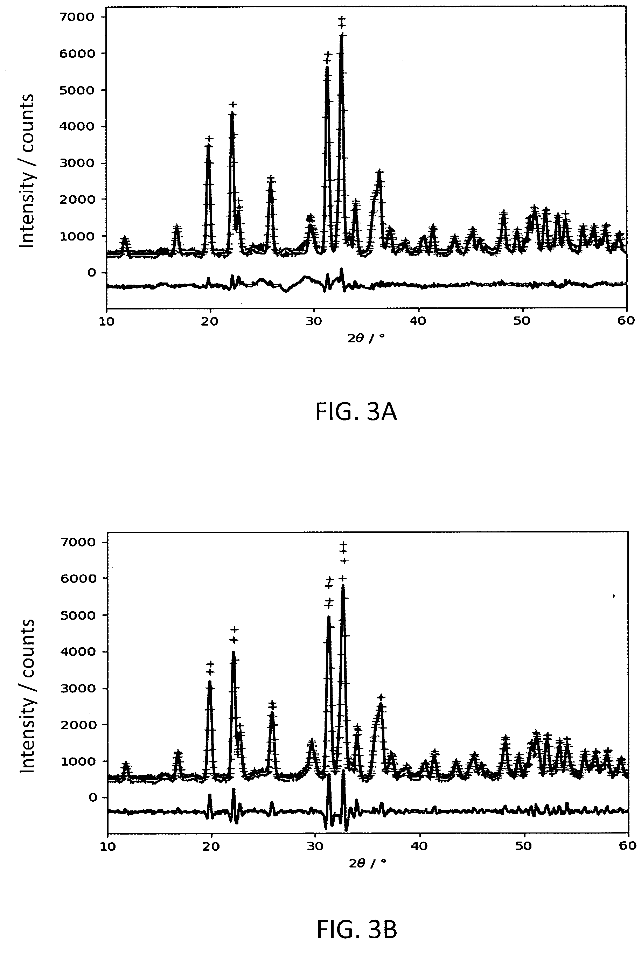

To illustrate the goodness-of-fit values, Figures 3A and 3B represent fitted diffraction patterns for the first structural model ($Fe_2TeO_5$ + $Fe_2TeO_6$ + $Fe_2O_3$) and the second structural model ($Fe_2TeO_5$ + $FeTeO_5$ + $Fe_2O_3$), respectively. In each figure, the obtained diffraction pattern (i.e., from step 101) is represented by the series of data points (the '+' symbols), while the predicted diffraction pattern is represented by the solid line overlaying the data points. Beneath each diffraction pattern is a line representing the error between the obtained and predicted diffraction patterns. As illustrated by the error line, the goodness-of-fit is better for the first structural model (Figure 3A) than the second structural model (Figure 3B) - this results in the lower S value of 2.28 for the first structural model compared to 2.35 for the second structural model.

With continued reference to the example shown in Figure 2, the first measured phase distribution matches the predicted phase distribution with respect to compounds $Fe_2TeO_5$ and $Fe_2O_3$, such that there is no error generated. However, the first measured phase distribution identifies $Fe_2TeO_6$ instead of $FeTeO_5$ which produces a squared error as shown in the fourth column. In contrast, the second measured phase distribution perfectly matches the predicted phase distribution (i.e. by identifying $FeTeO_5$ instead of $Fe_2TeO_6$) such that the squared error is zero for all compounds. Therefore, when the errors are summed and square rooted, the error in Measurement 1 (0.071) is higher than the error in Measurement 2 (0). In order to obtain a likelihood estimator based on the root mean square error, a scale factor is applied. In the example shown, a scale factor (k) of 10 is applied to the root mean square errors to produce values of 0.71 and 0 for

the likelihood estimators ($I_p$) of the first and second measured phase distributions, respectively.

The method 100 then includes modifying 107 the goodness-of-fit value based on the likelihood estimator. Therefore, in this example, the modified goodness-of-fit value (S') for the first measured phase distribution is 2.99 (i.e., an S value of 2.28 plus a likelihood estimator of 0.71) and for the second measured phase distribution is 2.35 (i.e., an S value of 2.35 plus a likelihood estimator of 0). Therefore, the method 100 is configured to proceed with the minimisation step 108 based on the second structural model in preference to the first structural model. This is because the likelihood estimator of the second structural model has biased the goodness-of-fit thereof to a lower value based on the better likelihood that the structural model exists, which in turn is based on a better match between the second measured phase distribution and a predicted phase distribution (based on, for example, the reaction conditions). Upon closer inspection of the fitted diffraction patterns in Figures 3A and 3B, it can be seen that although the average error is higher for the second structural model (which is mostly due to the errors in the large peaks), the qualitative fit of the line to the data points is more accurate, especially when considering some of the less intense peaks. This demonstrates how using a modified goodness-of-fit value as disclosed herein can increase the accuracy of materials characterisation in an automated Rietveld refinement method.

Although calculations for additional structural models are not illustrated in Figure 2, the method 100 further includes minimising 108 the goodness-of-fit value by repeating the process with other structural models. This repetition of steps may be the same as typical Rietveld refinement, where the structure is further refined by varying a range of parameters (e.g., lattice parameters) to generate additional structural models, which can be evaluated using the method steps 103-107 described above. Eventually, the Rietveld refinement settles and cannot be further refined. At this stage, the method 100 outputs 109 the optimal structural model based on the optimisation.

## Computer System

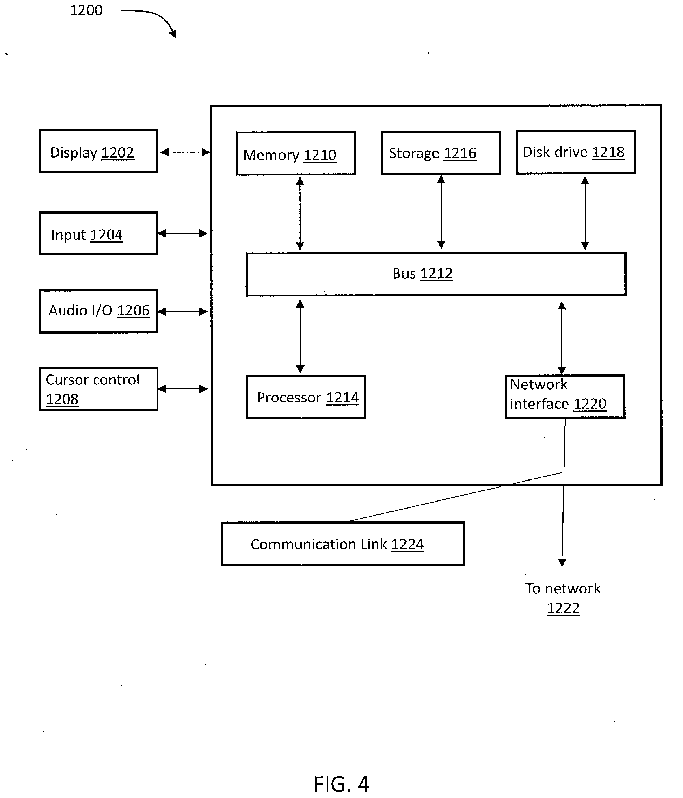

Figure 4 is a block diagram of a computer system 1200 suitable for implementing one or more embodiments of the present disclosure, including for example the methods described in relation to Figures 1-3B.

The computer system 1200 includes a bus 1212 or other communication mechanism for communicating information data, signals, and information between various components of the computer system 1200. The components include an input/output (I/O) component 1204 that processes a user (i.e., sender, recipient, service provider) action, such as selecting keys from a keypad/keyboard, selecting one or more buttons or links, etc., and sends a corresponding signal to the bus 1212. It may also include a camera for obtaining image data. The I/O component 1204 may also include an output component, such as a display 1202 and a cursor control 1208 (such as a keyboard, keypad, mouse, etc.). The display 1202 may be configured to present a login page for logging into a user account, and is configured to display a user interface. An optional audio input/output component 1206 may also be included to allow a user to use voice for inputting information by converting audio signals. The audio I/O component 1206 may allow the user to hear audio. A transceiver or network interface 1220 transmits and receives signals between the computer system 1200 and other devices, such as another user device, a merchant server, or a service provider server via network 1222. In one embodiment, the transmission is wireless, although other transmission mediums and methods may also be suitable. A processor 1214, which can be a micro-controller, digital signal processor (DSP), or other processing component, processes these various signals, such as for display on the computer system 1200 or transmission to other devices via a communication link 1224. The processor 1214 may also control transmission of information, such as cookies or IP addresses, to other devices.

The components of the computer system 1200 also include a system memory component 1210 (e.g., RAM), a static storage component 1216 (e.g., ROM), and/or a disk drive 1218 (e.g., a solid-state drive, a hard drive). The computer system 1200 performs specific operations by the processor 1214 and other components by executing one or more sequences of instructions contained in the system memory component 1210. For example, the processor 1214 can run the method 100 described above.

Logic may be encoded in a computer readable medium, which may refer to any medium that participates in providing instructions to a processor for execution. Such a medium may take many forms, including but not limited to, non-volatile media, volatile media, and transmission media. In various implementations, non-volatile media includes optical or magnetic disks, volatile media includes dynamic memory, such as a system memory component, and transmission media includes coaxial cables, copper wire, and fibre optics. In one embodiment, the logic is encoded in non-transitory computer readable medium. In one example, transmission media may take the form of acoustic or light waves, such as those generated during radio wave, optical, and infrared data communications.

Some common forms of computer readable media include, for example, floppy disk, flexible disk, hard disk, magnetic tape, any other magnetic medium, CD-ROM, any other optical medium, punch cards, paper tape, any other physical medium with patterns of holes, RAM, PROM, EPROM, FLASH-EPROM, any other memory chip or cartridge, or any other medium from which a computer is adapted to read.

In various embodiments of the present disclosure, execution of instruction sequences to practice the present disclosure may be performed by a computer system. In various other embodiments of the present disclosure, a plurality of computer systems coupled by a communication link to a network (e.g., such as a LAN, WLAN, PTSN, and/or various other wired or wireless networks, including telecommunications, mobile, and cellular phone networks) may perform instruction sequences to practice the present disclosure in coordination with one another.

It will also be understood that aspects of the present disclosure may be implemented using hardware, software, or combinations of hardware and software. Also, where applicable, the various hardware components and/or software components set forth herein may be combined into composite components comprising software, hardware, and/or both without departing from the spirit of the present disclosure. Where applicable, the various hardware components and/or software components set forth herein may be separated into subcomponents comprising software, hardware, or both without departing from the scope of the present disclosure. In addition, where applicable, it is contemplated that software components may be implemented as hardware components and vice-versa.

Software in accordance with the present disclosure, such as program code and/or data, may be stored on one or more computer readable mediums. It is also contemplated that software identified herein may be implemented using one or more general purpose or specific purpose computers and/or computer systems, networked and/or otherwise. Where applicable, the ordering of various steps described herein may be changed, combined into composite steps, and/or separated into sub-steps to provide features described herein.

The various features and steps described herein may be implemented as systems comprising one or more memories storing various information described herein and one or more processors coupled to the one or more memories and a network, wherein the one or more processors are operable to perform steps as described herein, as non-transitory machine-readable medium comprising a plurality of machine-readable instructions which, when executed by one or more processors, are adapted to cause the one or more processors to perform a method comprising steps described herein, and methods performed by one or more devices, such as a hardware processor, user device, server, and other devices described herein.

For example, the processor 1214 may comprise data ingestion circuitry. The step of obtaining 101 a diffraction pattern and/or diffraction data may be performed by the data ingestion circuitry. The processor 1214 may further comprise phase distribution prediction circuitry. The step of predicting 11 a phase distribution may be performed by the method 100 and may be performed by the phase distribution prediction circuitry. The processor 1214 may comprise refinement circuitry. The step of performing modified Rietveld refinement may be performed by the refinement circuitry. The controller 1214 may comprise optimisation circuitry. The step of optimising 108 the modified goodness-of-fit value may be performed by the optimisation circuitry. The step of outputting 109 a characterisation of the crystalline material based on the structural model may be performed using the user interface, for example on the display 1202.

As utilized herein, terms "component," "system," "interface," "unit" and the like are intended to refer to a computer-related entity, hardware, software (e.g., in execution), and/or firmware. For example, a component can be a processor, a process running on a processor, an object, an executable, a program, a storage device, and/or a computer. By way of illustration, an application running on a server and the server can be a component.

One or more components can reside within a process, and a component can be localized on one computer and/or distributed between two or more computers.

Further, these components can execute from various computer readable media having various data structures stored thereon. The components can communicate via local and/or remote processes such as in accordance with a signal having one or more data packets (e.g., data from one component interacting with another component in a local system, distributed system, and/or across a network, e.g., the Internet, a local area network, a wide area network, etc. with other systems via the signal).

It will be appreciated from the above description that many features of the different examples are interchangeable and combinable. The disclosure extends to further examples comprising features from different examples combined together in ways not specifically mentioned. Indeed, there are many features presented in the above examples and it will be apparent to the skilled person that these may be advantageously combined with one another.

## Claims

1. A computer implemented method of characterising a crystalline material, the method comprising:

   obtaining diffraction data from diffraction of the crystalline material;
   obtaining a predicted phase distribution of the crystalline material;
   performing modified Rietveld refinement on the diffraction data, wherein performing modified Rietveld refinement comprises:

   i) determining an R-weighted pattern value, $R_{wp}$, and an R-expected value, $R_e$, for a structural model of the crystalline material; and

ii) determining a goodness-of-fit value based on $R_{wp}$, $R_e$ and a likelihood estimator for the structural model;

optimising the goodness-of-fit value by repeating steps i) and ii) for different structural models of the crystalline material;
obtaining an optimised structural model that provides an optimised goodness-of-fit value;
outputting a characterisation of the crystalline material based on the obtained optimised structural model;

wherein the likelihood estimator is based on:

the predicted phase distribution of the crystalline material; and
a measured phase distribution, wherein the measured phase distribution is based on the structural model.

2. The method of claim 1, wherein the predicted phase distribution is based on synthesis information of the crystalline material.

3. The method of claim 1 or claim 2, wherein the predicted phase distribution is based on at least one of: ratios of precursor compositions; thermodynamic stability of precursor compositions; reaction temperature; reaction time; reaction atmosphere; reaction heating and cooling rates; applied fields; and instrumentation.

4. The method of any preceding claim, wherein the diffraction data is from X-ray diffraction of the crystalline material.

5. The method of any preceding claim, wherein the predicted phase distribution is based on at least one of: a graph network, an autoencoder, and a regression algorithm.

6. The method of any preceding claim, wherein the predicted phase distribution comprises fractions of expected phases.

7. The method of any preceding claim, wherein the predicted phase distribution is based on a machine learning model.

8. The method of claim 7, wherein the machine learning model is trained to predict a phase distribution based on input data comprising synthesis information.

9. The method of any preceding claim, wherein the goodness-of-fit value is based on a ratio of $R_{wp}$ to $R_e$.

10. The method of claim 9, wherein the goodness-of-fit value is based on adding the likelihood estimator to the ratio.

11. The method of any preceding claim, wherein the likelihood estimator is based on an error between the predicted phase distribution and the measured phase distribution.

12. The method of any preceding claim, wherein the likelihood estimator is based on at least one of: a root mean square error metric and a Kullback-Leibner divergence metric.

13. A computer program product comprising program instructions configured to program a programmable processor to perform the method of any of claims 1 to 12.

14. Programmable logic configured to perform the method of any of claims 1 to 12.

FIG. 1

| j | p | Measurement 1 | | Measurement 2 | |
|---|---|---|---|---|---|
| | | q1 | (p-q1)^2 | q2 | (p-q2)^2 |
| Fe2TeO5 | 93% | 93% | 0.00 | 93% | 0.00 |
| FeTeO5 | 5% | 0% | 0.0025 | 5% | 0.00 |
| Fe2TeO6 | 0% | 5% | 0.0025 | 0% | 0.00 |
| Fe2O3 | 2% | 2% | 0.00 | 2% | 0.00 |
| | | | | | |
| RMS | | 0.071 | | 0.000 | |
| S | | 2.28 | | 2.35 | |
| lp | | 0.71 | | 0.00 | |
| S' | | 2.99 | | 2.35 | |

FIG. 2

FIG. 3A

FIG. 3B

1200

FIG. 4

| | Europäisches Patentamt / European Patent Office / Office européen des brevets | **EUROPEAN SEARCH REPORT** | **Application Number** EP 24 31 5415 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DAVID W. I.: "Robust Rietveld refinement in the presence of impurity phases", JOURNAL OF APPLIED CRYSTALLOGRAPHY, vol. 34, no. 6, 1 December 2001 (2001-12-01), pages 691-698, XP093249557, DK ISSN: 0021-8898, DOI: 10.1107/S0021889801011396 * abstract * * page 691, left-hand column, paragraph 1 - page 692, right-hand column, paragraph 3 * * Equation (6) * * figure 5 * ----- | 1-14 | INV. G01N23/2055 G16C60/00 |
| Y | KISI E H: "RIETVELD ANALYSIS OF POWDER DIFFRACTION PATTERNS", MATERIALS FORUM, PERGAMON PRESS, OXFORD, GB, vol. 18, 1 January 1994 (1994-01-01), pages 135-153, XP002056617, ISSN: 0160-7952 * abstract * * page 135, left-hand column, paragraph 1 - page 136, left-hand column, paragraph 1 * * page 140, left-hand column, paragraph 2 - page 141, right-hand column, paragraph 1 * * page 144, left-hand column, paragraph 1-2 * * page 147, right-hand column, paragraph 4 * * page 149, left-hand column, paragraph 2 - right-hand column, paragraph 1 * * Equations (6) to (11) * ----- -/-- | 1-14 | TECHNICAL FIELDS SEARCHED (IPC) G01N G16C |

The present search report has been drawn up for all claims

| Place of search | Date of completion of the search | Examiner |
|---|---|---|
| The Hague | 14 February 2025 | Couteau, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

| | Europäisches Patentamt European Patent Office Office européen des brevets | **EUROPEAN SEARCH REPORT** | **Application Number** EP 24 31 5415 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DAVID W I. F.: "Powder Diffraction: Least-Squares and Beyond", JOURNAL OF RESEARCH OF THE NATIONAL INSTITUE OF STANDARDS AND TECHNOLOGY, vol. 109, no. 1, 1 February 2004 (2004-02-01), pages 107-123, XP093249479, DOI: 10.6028/jres.109.008 * abstract * * page 108, left-hand column, paragraph 2 - page 110, right-hand column, paragraph 1 * * figure 1 * ----- | 1-14 | |
| A | KEMETHMÜLLER STEFAN ET AL: "Quantitative Analysis of Crystalline and Amorphous Phases in Glass-Ceramic Composites Like LTCC by the Rietveld Method", JOURNAL OF THE AMERICAN CERAMIC SOCIETY, vol. 89, no. 8, 13 June 2006 (2006-06-13), pages 2632-2637, XP093249192, US ISSN: 0002-7820, DOI: 10.1111/j.1551-2916.2006.01113.x * abstract * * page 2633, left-hand column, paragraph 2 - right-hand column, paragraph 1 * ----- | 1-14 | TECHNICAL FIELDS SEARCHED (IPC) |

The present search report has been drawn up for all claims

| Place of search | Date of completion of the search | Examiner |
|---|---|---|
| The Hague | 14 February 2025 | Couteau, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)